# EUROPÄISCHE PATENTSCHRIFT

(11) **EP 2 642 961 B1**
(45) Veröffentlichungstag und Bekanntmachung des Hinweises auf die Patenterteilung: **08.04.2015**
(21) Anmeldenummer: 11791210.5
(22) Anmeldetag: 18.11.2011
(51) Int. Cl.: A61F 13/15, A61F 13/56, A61F 13/64

(54) **GEFALTETER INKONTINENZARTIKEL UND VERFAHREN ZUM HERSTELLEN DAVON**
FOLDED INCONTINENCE ARTICLE AND METHOD FOR PRODUCTION THEREOF
ARTICLE PLIÉ POUR INCONTINENCE ET SON PROCÉDÉ DE FABRICATION

(30) Priorität: 23.11.2010 DE 102010052264
(43) Veröffentlichungstag der Anmeldung: 02.10.2013
(73) Patentinhaber: Paul Hartmann AG, 89522 Heidenheim (DE)
(72) Erfinder: HOMANN, Vanessa, 22459 Hamburg (DE); KESSELMEIER, Rüdiger, 89542 Herbrechtingen (DE)
(86) Internationale Anmeldenummer: PCT/EP2011/005831
(87) Internationale Veröffentlichungsnummer: WO 2012/069166

(56) Entgegenhaltungen:
- DE-A1-102004 021 353
- US-A1- 2001 034 512

## Beschreibung

Die vorliegende Erfindung betrifft gefaltete, absorbierende Inkontinenzartikel des offenen Typs für inkontinente Erwachsene, und ein Verfahren zur Faltung absorbierender Inkontinenzartikel.

DE-102005035544-A1 beschreibt bereits einen Inkontinenzartikel mit an Seitenrändern des Hauptteils angestückten Materialabschnitten, die häufig auch als Windelohren bezeichnet werden, die im Randbereich Verschlusselemente tragen, wobei die Windelohren um wenigstens zwei Falzlinien auf sich selbst gefaltet sind und auf die körperzugewandte Seite des Hauptteils eingeschlagen sind, um eine übereinander eingeschlagene Anordnung zu bilden, die an einer ersten Fügestelle lösbar fixiert ist.

EP-1005316-B1 beschreibt die z-förmige Faltung der Windelohren einer Windel des offenen Typs, bei der zunächst das äußere Ende der Ohren nach hinten um mindestens die Verschlussflächenbreite gefaltet wird und anschließend in dieser Konfiguration nach vorne um die doppelte Verschlussflächenbreite zurückgefaltet wird, so dass die Verschlusselemente in dieser gefalteten Konfiguration nicht von einer Materiallage verdeckt sind und für den Anwender sofort sichtbar sind.

EP-1166735-B1 beschreibt ebenfalls eine z-förmige bzw. invers z-förmige Faltung der hinteren Windelohren, wobei die erste Faltung nach innen auf das Windelchassis erfolgt und anschließend das freie Ende des Seitenteils so ein- oder mehrfach nach außen umgefaltet wird, dass dieses im gefalteten Zustand nach außen weist und sich beim Anlegen der Windel - insbesondere an bettlägerige Personen - leicht entfalten lässt.

WO-2005/110321-A1 beschreibt die Faltung von absorbierenden Hygieneartikeln entlang erster und zweiter longitudinaler Falzlinien, derart, dass die Außenkanten der Windelohren über die Falzlinie hinaus reichen, so dass der gefaltete Artikel leicht zu entfalten ist, da die die Verschlusselemente tragende Außenkante direkt zu erreichen ist.

WO-2007/058761-A1 beschreibt die Herstellung von absorbierenden Hygieneartikeln mit separat angefügten Windelohren bei hohen Maschinengeschwindigkeiten ohne dass der Artikel oder die Verschlusselemente unbeabsichtigt zerknittert oder gefaltet werden, dadurch dass eine Faltung vorgenommen wird, die die Windelohren und Verschlusselemente schützt. Dazu wird das Windelohr, das auf seiner im angelegten Zustand nach innen liegenden Seite ein Verschlusselement trägt, an einer Längsachse auf die Innenseite des Chassis umgeschlagen und anschließend wird an einer zweiten Längsachse der gesamte Seitenteil auf das Chassis umgeschlagen, wobei die zweite Faltbreite mindestens so groß ist, wie die erste, damit der erste nach innen geschlagene Bereich durch die zweite Faltung nicht erneut gefaltet wird.

DE-102004021353-A1 offenbart einen absorbierenden Inkontinenzartikel mit einem Hauptteil, bestehend aus einem Vorderbereich, einem Rückbereich und einem in Längsrichtung dazwischen liegenden zwischen den Beinen eines Benutzers zu liegen kommenden Schrittbereich, wobei der Hauptteil einen Saugkörper umfasst, und mit an den Rückbereich angefügten hinteren Materialabschnitten und mit an den Vorderbereich angefügten vorderen Materialabschnitten, wobei sich die hinteren und vorderen Materialabschnitten in Querrichtung über seitliche Längsränder des Hauptteils hinaus erstrecken, wobei die Materialabschnitte eine im Anwendungszustand auf der körperzugewandten Seite liegende Innenseite und eine im Anwendungszustand auf der körperabgewandten Seite liegende Außenseite haben und wobei hintere Materialabschnitte an einem äußeren Randbereich der hinteren Materialabschnitte Verschlusselemente aufweisen, wobei hintere und vordere Materialabschnitte den Vorderbereich und den Rückbereich im Anwendungszustand des Artikels miteinander verbinden und wobei die Materialabschnitte vor Ingebrauchnahme des zusammengefalteten Artikels auf sich selbst und nach innen auf die körperzugewandte Seite des Rückbereichs des Hauptteils gefaltet sind.

Ausgehend von diesem Stand der Technik besteht das Problem der gefalteten Inkontinenzartikel darin, dass bei hohen Fertigungsgeschwindigkeiten im Herstellungsprozess der Anteil nicht spezifkationskonform hergestellter Produkte steigt. Als nicht spezifikationskonform hergestellt werden Produkte bezeichnet, die nicht die herstellerseitig festgelegten Vorgaben bzgl. der Qualität der Produkte erfüllen. Die Produktqualität wird bei Erhöhung der Fertigungsgeschwindigkeit dadurch beeinträchtigt, dass sich die auf den gefalteten Windelohren angebrachten Verschlusselemente während der Fertigung ungewollt öffnen. Wird der Inkontinenzartikel mitsamt den gefalteten Windelohren und den ungewollt geöffneten Verschlusselementen im nächsten Fertigungsschritt längs- und/oder quer gefaltet, so kommt das geöffnete oder teilgeöffnete Verschlusselement in Kontakt mit anderen Materiallagen des Inkontinenzartikels und wird auf diesen ungewollt fixiert. Dies führt dazu, dass der Inkontinenzartikel nicht mehr in gewohnter Weise entfaltet werden kann und gegebenenfalls die Verschlusselemente für eine sinngemäße Fixierung nicht mehr brauchbar sind. Würden derartige Inkontinenzartikel nicht aus dem Produktionsprozess entfernt, so würde die ungewollte Fixierung bei der anschließenden Verpackung der Inkontinenzartikel unter Druck in einen Kunststofffolienbeutel weiter verfestigt, so dass derartige Produkte unbrauchbar werden, da sie sich nicht mehr einwandfrei öffnen und anlegen lassen.

Die Aufgabe der vorliegenden Erfindung besteht darin, absorbierende Inkontinenzartikel des offenen Typs mit angefügten Windelohren bei hoher Fertigungsgeschwindigkeit ohne Beeinträchtigung der Funktionalität der Verschlussmittel produzieren zu können. Die Inkontinenzartikel sollen außerdem in einer benutzerfreundlichen Anordnung zur Verfügung gestellt werden.

Diese Aufgabe wird bei einem Inkontinenzartikel des offenen Typs mit an einen Seitenrandabschnitt des Hauptteils im Rückbereich angefügten hinteren Materialabschnitten und im Vorderbereich angefügten vorderen Materialabschnitten (Windelohren, Seitenklappen oder -teile) erfindungsgemäß dadurch gelöst, dass vor Ingebrauchnahme des Inkontinenzartikels die hinteren Materialabschnitte, deren Randbereich ein Verschlusselement aufweist, das eine Verschlusselementlasche mit einem freien Fingerlift umfasst, welche vor Gebrauch auf die Innenseite des Materialabschnitts zurückgefaltet ist, auf sich selbst gefaltet sind an zu der Längsrichtung parallel verlaufenden Falzlinien unter Bildung von inneren Teilabschnitten, mittleren Teilabschnitten und äußeren Teilabschnitten, wobei die inneren Teilabschnitte an den jeweiligen Seitenrandabschnitt des Hauptteils angefügt sind und die äußeren Teilabschnitte den Längsrandbereich umfassen und die mittleren Teilabschnitte auf die Innenseite der äußeren Teilabschnitte gefaltet sind, derart dass die mittleren Teilabschnitte den freien Fingerlift der Verschlusselementlasche vollflächig überfangen und dass die derart gefalteten hinteren Materialabschnitte an Einschlagachsen nach innen auf die Innenseite des Rückbereichs des Hauptteils zu einer eingeschlagenen Anordnung eingeschlagen sind, wobei in der eingeschlagenen Anordnung die Innenseite des Randbereichs von der Innenseite des Rückbereichs des Hauptteils abgewandt orientiert ist.

Die Verschlusselementlaschen wirken zum Schließen des Inkontinenzartikels in Gebrauch mit einer Außenseite des Vorderbereichs des Hauptteils und/oder der Materialabschnitte im Vorderbereich lösbar haftend zusammen. Hierzu können die Verschlusselementlaschen beispielsweise eine Haftkleberzone oder mechanische Verschlusshilfen wie Kletthaken aufweisen.

Die Erfinder haben erkannt, dass der Auslöser für das ungewollte Öffnen der Verschlusselementlaschen im Fertigungsprozess eine Luftzug-Kraft ist, die auf die Verschlusselementlaschen wirkt und die mit steigender Fertigungsgeschwindigkeit ansteigt. Die Gefahr des Einwirkens dieser Luftzug-Kraft ist insbesondere zu dem Zeitpunkt gegeben, zu dem die hinteren Materialabschnitte in der gefalteten Anordnung bereits auf die Innenseite des Hauptteils eingeschlagen sind und insbesondere unmittelbar bevor oder während der Inkontinenzartikel anschließend quer zu seiner Längsrichtung gefaltet wird. Wenn diese Luftzug-Kraft die Haftkraft der Verschlusselementlasche auf der Innenseite des Materialabschnitts, auf die es zurückgeschlagen wurde, übersteigt, so öffnet sich das Verschlusselement ungewollt. Wie oben beschrieben sind derartige Inkontinenzartikel häufig völlig unbrauchbar.

Die Erfinder haben weiterhin erkannt, dass die Verschlusselemente durch die erfindungsgemäße Anordnung gegen die auftretende Luftzug-Kraft im Fertigungsprozess weitestgehend geschützt sind. Dies wird anhand der Figuren noch näher erläutert werden.

Ferner haben die Erfinder in einer bevorzugten Ausführungsform des erfindungsgemäßen Inkontinenzartikels ein Produkt bereitgestellt, welches sich durch die Anordnung der äußeren Längsrandbereiche der hinteren Materialabschnitte derart, dass diese von der Produktlängsmittelachse abgewandt in Richtung der äußeren seitliche Längsränder des Hauptteils orientiert sind, vom Benutzer komfortabel entfalten lässt.

Damit der Benutzer die hinteren Materialabschnitte als solche erkennt und komfortabel ergreifen und entfalten kann, sind die Verschlusselementlasche des absorbierenden Inkontinenzartikels vorzugsweise zu mindestens 25 % und höchstens 90 % ihrer Erstreckung in Querrichtung, vorzugsweise zu mindestens 40 % und höchstens zu 75 % ihrer Erstreckung in Querrichtung von mittleren Teilabschnitten überdeckt oder die mittleren Teilabschnitte überdecken die Verschlusselementlasche um mindestens 10 mm und höchstens 50 mm, weiterhin bevorzugt mindestens 20 mm und höchstens 40 mm in Querrichtung.

Vorzugsweise steht ein äußerer Seitenrand des äußeren Teilabschnitts eines hinteren Materialabschnitts gegenüber den darüber gefalteten mittleren Teilabschnitten in Querrichtung um das Maß D über.

Das Maß D beträgt vorzugsweise mindestens 10 % und höchstens 85 %, bevorzugt mindestens 40 % und höchstens 60 %, der Breite L5 jedes äußeren Teilabschnittes. Alternativ beträgt der Überstand in Querrichtung um das Maß D vorzugsweise mindestens 5 mm und höchstens 50 mm, weiterhin bevorzugt mindestens 24 mm und höchstens 36 mm.

Die hinteren Materialabschnitte sind vorzugsweise derart auf sich selbst gefaltet, dass sie durch die Faltung in jeweils vier Teilabschnitte unterteilt werden, wobei die Breiten L3, L4 der jeweilige mittleren Teilabschnitte kleiner sind als die Breiten L5 der jeweiligen äußeren Teilabschnitte. Weiterhin bevorzugt sind die Breiten L3, L4 der mittleren Teilabschnitte identisch und/oder das Verhältnis der Breiten L3, L4 der mittleren Teilabschnitte zu den Breiten L5 der äußeren Teilabschnitte liegt zwischen 1:1 und 1: 2,5.

Die Faltung der hinteren Materialabschnitte auf sich selbst erfolgt vorzugsweise derart, dass zunächst Außenseite auf Außenseite eines jeweiligen hinteren Materialabschnittes auf sich selbst gefaltet wird. Die so gebildete doppellagige Anordnung wird dann nochmals auf sich selbst gefaltet, derart, dass Innenseite auf Innenseite eines jeweiligen hinteren Materialabschnittes gefaltet wird. Es entsteht eine bereichsweise vierlagige Anordnung. Diese kann gegebenenfalls ein weiteres Mal auf sich selbst zu einer achtlagigen Anordnung gefaltet werden: Bei der Faltung der hinteren Materialabschnitte auf sich selbst schneiden Falzlinien vorzugsweise nicht die auf die Innenseite der Materialabschnitte zurück gefalteten Verschlusselementlaschen.

Die derart aufeinander gefalteten hinteren Materialabschnitte sind in der gefalteten Konfiguration vorzugsweise lösbar fixiert, insbesondere durch punktförmige durch Ultraschallschweißung erzeugte Fügestellen. Im Zusammenhang mit der lösbaren Fixierung der gefalteten Materialabschnitte wird auf DE102009022529.3 verwiesen.

Weiterhin erweist es sich als vorteilhaft, wenn an jedem der gefalteten hinteren Materialabschnitte an einem jeweiligen Längsrandbereich ein Anfassbereich vorhanden ist. Dieser zum manuellen Ergreifen des gefalteten Materialabschnitts geeignete Bereich kann den gesamten Längsrandbereich umfassen. Vorzugsweise umfasst der Anfassbereich einen Teil des Längsrandbereichs, der frei von Fügestellen ist, vorzugsweise einen in der Mitte des Längsrandbereichs liegenden Bereich. Es hat sich gezeigt, dass die lösbare Fixierung mittels Fügestellen so gestaltet sein kann, dass sich durch einmaliges Ziehen am jeweiligen Anfassbereich der jeweilige Materialabschnitt vollständig entfalten lässt, wobei vorzugsweise alle Fügestellen gelöst oder aufgetrennt werden.

In Weiterbildung der Erfindung erweist es sich als vorteilhaft, wenn die hinteren Materialabschnitte eine größere, vorzugsweise eine um mindestens 10%, insbesondere eine um mindestens 15% größere Flächenerstreckung (in cm²) aufweisen als die vorderen Materialabschnitte. Insbesondere kann die Länge L7 der hinteren Materialabschnitte, also deren Erstreckung in Windellängsrichtung mindestens 13 cm, weiter insbesondere mindestens 18 cm und weiter insbesondere mindestens 22 cm betragen. Es erweist sich weiterhin als vorteilhaft, wenn die Länge L7 der hinteren Materialabschnitte mindestens 10 %, insbesondere mindestens 15%, weiter insbesondere mindestens 20% und weiter insbesondere mindestens 22% der Gesamtlänge der Inkontinenzwegwerfwindel beträgt. Vorteilhafterweise beträgt die Gesamtlänge der Inkontinenzwegwerfwindel 50-120 cm, insbesondere 60-110 cm und weiter insbesondere 70-110 cm. Weiterhin erweist es sich als vorteilhaft, wenn die vorderen Materialabschnitte eine geringere, insbesondere eine um mindestens 5%, weiter insbesondere eine um mindestens 10%, weiter insbesondere eine um mindestens 15% und weiter insbesondere eine um höchstens 50% geringere Länge L6 aufweisen, als die hinteren Materialabschnitte. In Weiterbildung der Erfindung erweist es sich als vorteilhaft, wenn die Breite L1 der Materialabschnitte, also die Erstreckung der Materialabschnitte in Querrichtung über den Seitenrand des Windelhauptteils hinaus 12-40 cm, insbesondere 13-30 cm, weiter insbesondere 14-27 cm beträgt. Vorzugsweise weisen die vorderen Materialabschnitte die gleiche Breite auf wie die hinteren Materialabschnitte.

Vorteilhafterweise sind auch die vorderen Materialabschnitte vor Ingebrauchnahme des zusammengefalteten Artikels auf sich selbst gefaltet, vorzugsweise an zu der Längsrichtung parallel verlaufenden Falzlinien. Die Falzlinien unterteilen die vorderen Materialabschnitte solchenfalls vorzugsweise in jeweils vier Teilabschnitte, wobei die Breiten L3, L4 der mittleren Teilabschnitte kleiner sind als die Breite der äußeren Teilabschnitte L5. Vorzugsweise erfolgt die Faltung der vorderen Materialabschnitte auf sich selbst derart, dass zunächst Außenseite auf Außenseite eines jeweiligen vorderen Materialabschnittes auf sich selbst gefaltet wird. Die so gebildete doppellagige Anordnung wird dann nochmals auf sich selbst gefaltet, derart, dass Innenseite auf Innenseite eines jeweiligen vorderen Materialabschnittes gefaltet wird.

Es hat sich weiter als vorteilhaft erwiesen, die vorderen und/oder hinteren Materialabschnitte aus einem Vliesstoffmaterial zu bilden. Geeignet sind insbesondere alle Vliesstoffmaterialien, die zumindest eine Rezepturkomponente auf Basis eines thermoplastischen Polymers enthalten. Die Vliesstoffe können Fasern aus PE, PP, PET, Rayon, Cellulose, PA und Mischungen dieser Fasern enthalten. Auch Bi- oder Multikomponentenfasern sind denkbar und vorteilhaft. Vorteilhaft sind insbesondere Kardenvliese, Spinnvliese, wasserstrahlvernadelte Vliese, SM-Vliese, SMS-Vliese, SMMS-Vliese oder auch Laminate aus einer oder mehreren dieser Vliesarten, wobei S für Spunbond- und M für Meltblown-Vliesschichten steht. Besonders bevorzugt sind Spinnvliese, da diese eine hohe Festigkeit in Längs- und Querrichtung aufweisen und somit den auf sie durch das Eingreifen von gegebenenfalls vorhandenen mechanischen Verschlusshilfen einwirkenden Scherkräften besonders gut standhalten können. Um zu verhindern, dass beim Lösen der mechanischen Verschlusshilfen Fasern aus dem Vliesverbund herausgerissen werden, ist vorteilhaft, die Vliesstoffkomponente mit einem Prägemuster zu versehen, vermittels dessen vorzugsweise alle Fasern der Vlieskomponente gebunden sind. Vorteilhaft ist solchenfalls insbesondere ein Thermoprägemuster, das insbesondere vorteilhaft durch Kalandern des Vliesstoffes unter Zuführung von thermischer Energie erzeugt wird.

Zur Herstellung eines erfindungsgemäßen gefalteten Inkontinenzartikels werden die auf sich gefalteten hinteren Materialabschnitte an vorzugsweise durch den Windelhauptteil, vorzugsweise parallel zur Längsachse verlaufenden Einschlagachsen derart auf die Innenseite des Rückbereichs eingeschlagen, dass die Materialabschnitte mit einem geringen Abstand voneinander oder auf Stoß zu liegen kommen oder zumindest bereichsweise einander überlappend. Vorteilhaft könnte auch der Seitenrand des Hauptteils im Bereich der Anfügung der hinteren Materialabschnitte die Einschlagachsen bilden.

Entsprechend betrifft die Erfindung auch ein Verfahren zur Herstellung eines gefalteten Inkontinenzartikels wie vorstehend beschrieben, wobei die hinteren Materialabschnitte zunächst einmal parallel zur Längsachse auf sich selbst gefaltet werden und anschließend die so gebildete doppellagige Anordnung nochmals parallel zur Längsachse auf sich selbst gefaltet wird und dabei der freie Fingerlift der Verschlusselementlasche von mittleren Teilabschnitten vollflächig überfangen wird zur Bildung einer zumindest bereichsweise vierlagigen Anordnung. Die derart auf sich selbst gefalteten hinteren Materialabschnitte werden an den Einschlagachsen nach innen auf die Innenseite des Rückbereichs des Hauptteils eingeschlagen, derart, dass die Innenseite des Längsrandbereichs eines jeweiligen hinteren Materialabschnitts von der Innenseite des Rückbereichs des Hauptteils abgewandt orientiert ist.

Bevorzugt werden die Materialabschnitte zunächst auf sich selbst gefaltet, anschließend in gefalteter Konfiguration an einem ersten und einem zweiten Seitenrandabschnitt am Rückbereich des Hauptteil fixiert und dann auf die Innenseite des Hauptteils eingeschlagen.

In Weiterbildung dieses Erfindungsgedankens wird der Inkontinenzartikel mindestens einmal, vorzugsweise zweimal nach innen, vorzugsweise an in Querrichtung verlaufenden Faltlinien, auf sich selbst gefaltet, vorzugsweise derart, dass zunächst der Vorderbereich nach innen auf die Innenseite des Hauptteils und anschließend der Rückbereich auf den Vorderbereich gefaltet wird. Dadurch entsteht ein im Herstell- und Verpackungsprozess aufgrund seiner kompakten Größe gut handhabbares Produkt, dessen Sichtseiten in der zusammengefalteten Konfiguration durch die auch im Anwendungszustand die äußerste Lage bildende Materiallage gebildet wird, so dass die Innenseite des Artikels vor Gebrauch vor Verunreinigung geschützt ist.

Vorzugsweise erfolgt die Förderung der Inkontinenzartikel im Zuge der Herstellung in einer Fertigungsmaschine, insbesondere nach dem Verfahrensschritt des Faltens und Einschlagens der hinteren Materialabschnitte an den Einschlagachsen nach innen auf die körperzugewandte Seite des Rückbereichs des Hauptteils, parallel zur Längsrichtung und mit einer Bahngeschwindigkeit von mehr als 200 m/min, insbesondere mehr als 250 m/min, weiter insbesondere von mehr als 300 m/min, weiter insbesondere von mehr als 350 m/min. Vorzugsweise werden die Inkontinenzartikel hierbei bis einschließlich dieses vorgenannten Verfahrensschrittes noch endlos, also noch mit ihrem jeweiligen späteren Hüftöffnungsrändern aneinander verbunden gefördert. Vorzugsweise erst in einem nachgelagerten Verfahrensschritt werden die Inkontinenzartikel in Querrichtung zur Bildung der vereinzelten Inkontinenzartikel voneinander getrennt und nachfolgend wie zuvor beschrieben an in Querrichtung verlaufenden Faltlinien auf sich selbst gefaltet.

Weitere Merkmale, Einzelheiten und Vorteile der Erfindung ergeben sich aus den beigefügten Patentansprüchen, der zeichnerischen Darstellung und nachfolgenden Beschreibung einer bevorzugten Ausführungsform der Erfindung. In der Zeichnung zeigt:
Figur 1 eine Draufsicht auf einen vollständig entfalteten Inkontinenzartikel
Figur 2 eine Teildraufsicht auf einen erfindungsgemäß gefalteten Inkontinenzartikel mit auf sich selbst gefalteten hinteren Materialabschnitten
Figur 2a eine Schnittansicht des Inkontinenzartikels nach Figur 2 (A-A)
Figur 3a und 3b zeigen Schnittansichten eines an den Randbereich eines hinteren Materialabschnitts angefügten Verschlusselementes
Figur 4a zeigt eine Schnittansicht eines hinteren Materialabschnittes vor der Faltung auf sich selbst und den Schritt der ersten Faltung auf sich selbst
Figur 4b zeigt eine Schnittansicht eines hinteren Materialabschnittes nach der ersten Faltung auf sich selbst und den Schritt der zweiten Faltung auf sich selbst
Figur 4c zeigt eine Schnittansicht eines hinteren Materialabschnittes nach der ersten und zweiten Faltung auf sich selbst

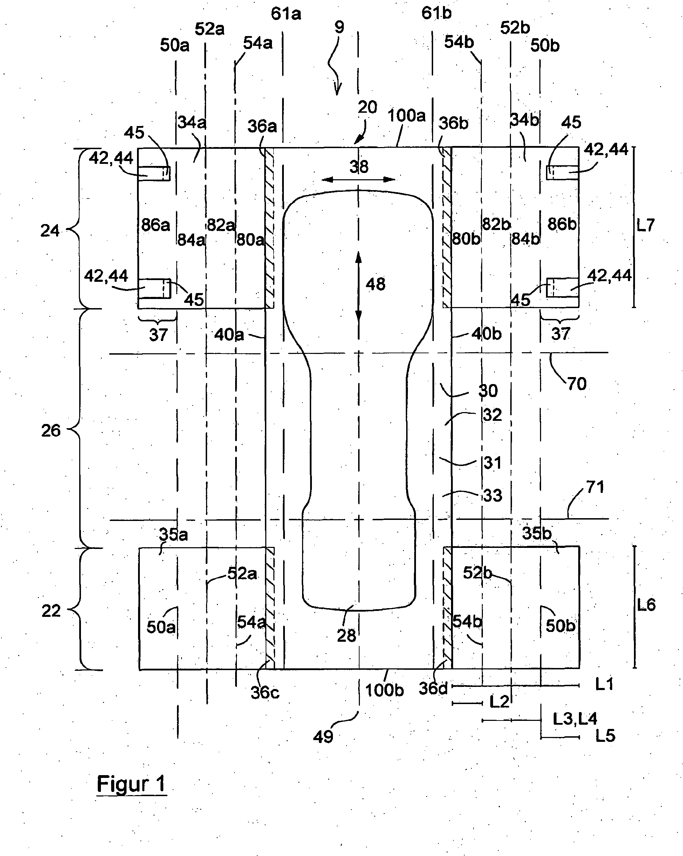

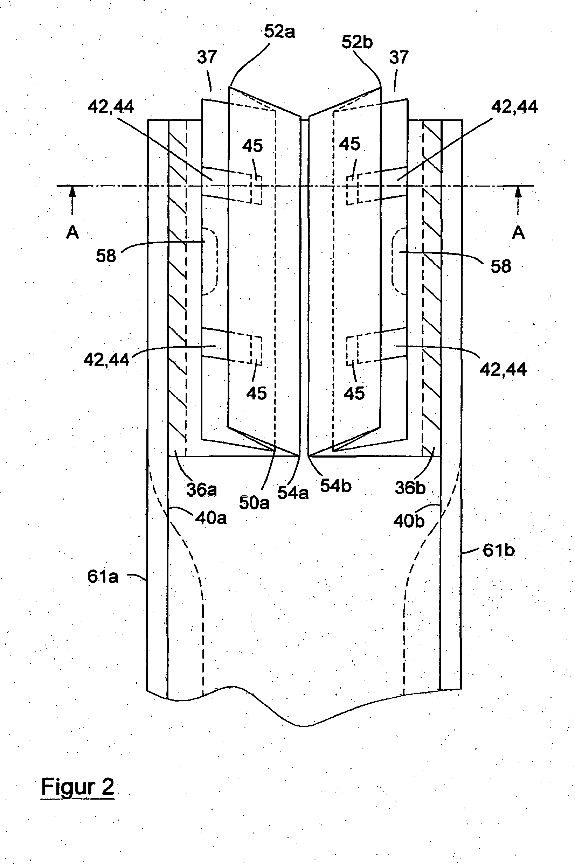

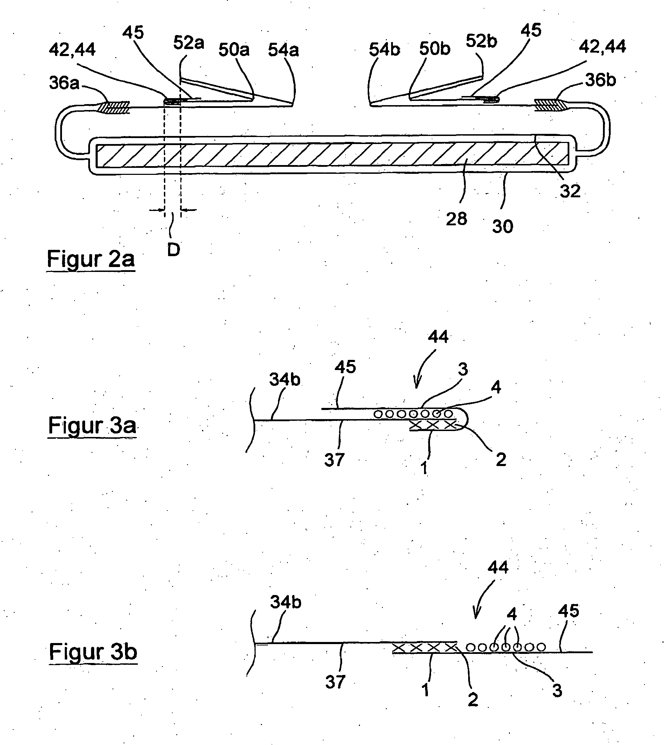

Ein erfindungsgemäßer Inkontinenzartikel 9 ist in den Figuren 1, 2 und 2a schematisch dargestellt. Er umfasst einen insgesamt mit dem Bezugszeichen 20 bezeichneten Hauptteil, der häufig auch als Chassis bezeichnet wird. Der Hauptteil 20 umfasst einen Vorderbereich 22, einen Rückbereich 24 und einen dazwischen liegenden Schrittbereich 26, der zwischen den Beinen eines Benutzers zu liegen kommt, wenn der Inkontinenzartikel 9 an einen Benutzer angelegt ist. Der Hauptteil 20 umfasst einen Saugkörper 28, der zur Aufnahme und dauerhaften Speicherung von Körperflüssigkeiten in geeigneter Weise dimensioniert ist. Der Saugkörper umfasst vorzugsweise Zellulosefasern und superabsorbierende Polymerpartikel (SAP) und ist von einer flüssigkeitsundurchlässigen Schicht 30 unterfangen, welche auch die äußere Sichtseite des Inkontinenzartikels 9 bilden kann. Oberhalb des Saugkörpers 28 kann ein flüssigkeitsdurchlässiges Topsheet 32 vorgesehen sein.

Im Rückbereich 24 sind an einem ersten Seitenrandabschnitt 36a des Hauptteils 20 ein erster eine hintere Seitenklappe oder Seitenabschnitt bildender Materialabschnitt 34a und an einem zweiten Seitenrandabschnitt 36b des Hauptteils 20 ein zweiter eine hintere Seitenklappe oder Seitenabschnitt bildender Materialabschnitt 34b überlappend angefügt (in Figur 1 schraffiert dargestellt). Die Materialabschnitte 34a, 34b weisen eine rechteckförmige Kontur auf. Es wären auch Materialabschnitte 34a, 34b, die eine Konturierung am Beinausschnitt aufweisen, denkbar und vorteilhaft, wie in DE-102007024180-A1 offenbart. Die äußeren Querränder von Hauptteil 20 und den hinteren Materialabschnitten 34a, 34b bilden den hinteren Hüftöffnungsrand 100a.

Die im Anwendungszustand dem Körper des Trägers abgewandte Seite des Inkontinenzartikels 9 wird als Außenseite 31 bezeichnet, die im Anwendungszustand dem Körper des Trägers zugewandte Seite des Inkontinenzartikels 9 als Innenseite 33. Nach diesem Verständnis ist in der eben ausgefalteten Konfiguration des Inkontinenzartikels 9 jeder Komponente des Inkontinenzartikels 9 eine Innenseite und eine Außenseite zuzuordnen.

Figur 1 zeigt eine Sicht auf die Innenseite 33 des Inkontinenzartikels 9 im vollständig entfalteten Zustand. Die hinteren Materialabschnitte 34a, 34b tragen jeweils zwei Verschlusselemente 42 mit auf sich selbst gefalteten und zum bestimmungsgemäßen Gebrauch entfaltbaren Verschlusselementlaschen 44, welche freie Fingerlifte 45 aufweisen. Denkbar wäre auch, die hinteren Materialabschnitte 34a, 34b mit nur jeweils einem Verschlusselement 42 auszustatten. Die Verschlusselementlaschen 44 wirken zum Schließen des Inkontinenzartikels in Gebrauch mit einer Außenseite 31 des Vorderbereichs 22 des Hauptteils 20 und/oder der Materialabschnitte im Vorderbereich 22 lösbar haftend zusammen. Hierzu können die Verschlusselementlaschen eine Haftkleberzone oder eine Zone mit mechanischen Verschlusshilfen wie Kletthaken aufweisen.

Im Vorderbereich 22 des Inkontinenzartikels sind ebenfalls auf sich selbst gefaltete Seitenabschnitte bildende Materialabschnitte 35a, 35b vorgesehen, die jedoch keine Verschlusselemente aufweisen. Die vorderen Materialabschnitte 35a, 35b weisen eine rechteckförmige Kontur auf. Es wären auch Materialabschnitte 35a, 35b, die eine Konturierung am Beinausschnitt aufweisen, denkbar und vorteilhaft, wie in DE-102007024180-A1 offenbart. Die äußeren Querränder von Hauptteil 20 und den vorderen Materialabschnitten 35a, 35b bilden den vorderen Hüftöffnungsrand 100b. Der jeweilige hintere Materialabschnitt 34a, 34b im Rückbereich 24 des Hauptteils ist, wie aus der Figur 2 ersichtlich, derart auf sich selbst gefaltet, das die mittleren Teilabschnitte 82a, 84a, 82b, 84b den freien Fingerlift 45 einer jeweiligen Verschlusselementlasche 44 vollflächig überfangen und die Anordnung der äußeren Längsrandbereiche 37 der hinteren Materialabschnitte derart ist, dass diese von der Produktlängsmittelachse 49 abgewandt in Richtung der äußeren seitliche Längsränder 40 des Hauptteils orientiert sind.

Im Zuge der Fertigung der Inkontinenzartikel 9 werden die jeweiligen hinteren Materialabschnitte 34a, 34b ausgehend von der Darstellung nach Figur 1 und nachdem sie auf sich selbst gefaltet wurden, nach innen an jeweiligen zur Längsrichtung 48 parallelen Einschlagachsen 61a, 61b, die in der dargestellten Ausführungsform innerhalb des Hauptteils 20 verlaufen, zu einer eingeschlagenen Anordnung umgeschlagen in die in Figur 2 dargestellte Position. In dieser Anordnung werden die Inkontinenzartikel 9 im Zuge der Fertigung insbesondere noch endlos in der Längsrichtung mit einer hohen Bahngeschwindigkeit, insbesondere mit einer Bahngeschwindigkeit von mehr als 200 m/min, weiter insbesondere mit einer Bahngeschwindigkeit von mehr als 300 m/min, weiter insbesondere mit einer Bahngeschwindigkeit von mehr als 350 m/min gefördert. Solchenfalls wirkt auf die Oberseite der Bahn ein starker Luftzug ("Fahrtwind") ein. Dies birgt die Gefahr, dass eine starke Luftzug-Kraft auf die die Oberseite der Bahn bildenden Komponenten ausgeübt wird.

Die Erfinder haben erkannt, dass die Verschlusselementlaschen in besonderem Maße der Gefahr des zu Fertigungsfehlern führenden Einwirkens starker Luftzug-Kräfte ausgesetzt sind: Die Verschlusselementlasche 44 ist mit ihrem Herstellerende 1 üblicherweise unlösbar, das heißt sehr fest vorzugsweise mittels eines Permanentklebstoffes 2 oder auch mittels Thermobonding oder Ultraschallschweißen oder anderen Fügemethoden an einem Randbereich 37 der hinteren Materialabschnitte 34a, 34b fixiert (Figur 3a). An ihrem Benutzerende 3 hingegen ist die Verschlusselementlasche 44 üblicherweise zum Zwecke der späteren Verwendung zerstörungsfrei ablösbar, also mit einer geringen Kraft vom Anwender in den Gebrauchszustand entfaltbar fixiert, vorzugsweise mittels eines Haftklebstoffes 4 und/oder mittels mechanischer Verschlusselemente wie Kletthaken. Figur 3b zeigt die bestimmungsgemäß unmittelbar vor Gebrauch des Inkontinenzartikels entfaltete Verschlusselementlasche 44 nach dem zerstörungsfreien Ablösen von der Innenseite 33 des Randbereiches 37 eines hinteren Materialabschnitts 34a, 34b. Außerdem weist die Verschlusselementlasche 44 an einem äußersten Bereich ihres Benutzerendes 3 einen so genannten Fingerlift 45 auf, das heißt einen Endabschnitt, welcher zum Zwecke des einfachen Ergreifens durch den Anwender mit einer noch geringeren Kraft ablösbar ist, oder vorzugsweise, wie im dargestellten Fall, gänzlich unfixiert verbleibt.

In der erfindungsgemäßen Anordnung ist sichergestellt, dass der Fingerlift 45 vollflächig von mittleren Teilabschnitte 82a, 84a, 82b, 84b überfangen wird und somit nicht zu den die Oberseite der Bahn bildenden Komponenten gehört und somit nicht oder weniger stark den oben beschriebenen Luftzug-Kräften ausgesetzt ist.

Vorzugsweise ist zusätzlich zu dem vollflächig überfangenen Fingerlift 45 die Verschlusselementlasche 44 zu mindestens 25 % und höchstens 90 % ihrer Erstreckung in Querrichtung 38, vorzugsweise zu mindestens 40 % und höchstens zu 75 % ihrer Erstreckung in Querrichtung 38 von mittleren Teilabschnitten 82a, 84a, 82b, 84b überdeckt. Alternativ überdecken die mittleren Teilabschnitte 82a, 84a, 82b, 84b die Verschlusselementlasche 44 um mindestens 10 mm und höchstens 50 mm, weiterhin bevorzugt mindestens 20 mm und höchstens 40 mm in Querrichtung 38 überdecken. Damit ist auf der einen Seite ein hohes Mass an Prozesssicherheit gewährleistet, derart dass verhindert wird, dass bei einem zu geringen Überfangen der Verschlusselementlasche 44 durch die mittleren Teilabschnitte 82a, 84a, 82b, 84b die Luftzug-Kraft auf die überfangenden Materiallagen einwirkt, diese gegebenenfalls verschiebt und in der Folge gegebenenfalls den Fingerlift 45 freilegt. Auf der anderen Seite ist in dieser Konfiguration das Verschlusselement 42 vom Benutzer gut zu erkennen und die Materialabschnitte lassen sich komfortable ergreifen und entfalten.

Dafür erweist es sich als vorteilhaft, wenn an jedem der hinteren Materialabschnitte 34a, 34b an einem jeweiligen Randbereich 37 ein Anfassbereich 58 vorhanden ist (Figur 2). Der Anfassbereich 58 ist der zum manuellen Ergreifen des gefalteten Materialabschnitts 34a, 34b geeignete Bereich, um diesen zu entfalten. Der jeweilige Anfassbereich 58 der Materialabschnitte 34a, 34b ist in der in Figur 2 dargestellten Konfiguration zu einer Produktlängsmittelachse 49 des Inkontinenzartikels 9 hin nach außen gewandt. Der Benutzer kann durch gleichzeitiges Greifen der Anfassbereiche 58 mit beiden Händen und einer Zugbewegung beider Hände nach außen das gefaltete Produkt komfortabel entfalten. Insbesondere entfällt solchenfalls die Notwendigkeit, die gefalteten Materialabschnitte 34a, 34b an den Einschlagachsen 61a, 61b zunächst auszuschlagen, bevor die gefalteten Materialabschnitte 34a, 34b mit einer Zugbewegung nach außen entfaltet werden können.

Weiterhin bevorzugt ist ein jeweiliger hinterer Materialabschnitt 34a, 34b derart gefaltet, dass ein äußerer Seitenrand 11 des äußeren Teilabschnitts 86a, 86b eines hinteren Materialabschnitts 34a, 34b gegenüber den darüber gefalteten mittleren Teilabschnitten 82a, 84a, 82b, 84b in Querrichtung 38 um das Maß D übersteht. Das Maß D beträgt vorzugsweise mindestens 10 % und höchstens 85 %, bevorzugt mindestens 40 % und höchstens 60 %, der Breite L5 jedes äußeren Teilabschnittes 86a, 86b. Alternativ beträgt der Überstand in Querrichtung 38 um das Maß D vorzugsweise mindestens 5 mm und höchstens 50 mm, weiterhin bevorzugt mindestens 24 mm und höchstens 36 mm.

Die aufeinander gefalteten Teilabschnitte 80a, 82a, 84a, 86a des Materialabschnittes 34a und die aufeinander gefalteten Teilabschnitte 80b, 82b, 84b, 86b des Materialabschnittes 34b sind in der gefalteten Konfiguration vorzugsweise lösbar fixiert, insbesondere durch punktförmige durch Ultraschallschweißung erzeugte Fügestellen, vorzugsweise mit einem Durchmesser von 0,35 mm und einer Fläche von 0,0962 mm². Es hat sich gezeigt, dass diese lösbare Fixierung so gestaltet sein kann, dass sich durch einmaliges Ziehen an dem jeweiligen ersten Anfassbereich 58 der jeweilige Materialabschnitt 34a, 34b vollständig entfalten lässt, wobei vorzugsweise alle Fügestellen gelöst oder aufgetrennt werden.

In der dargestellten bevorzugten Ausführungsform werden die in Längsrichtung aufeinander gefalteten hinteren Materialabschnitte 34a, 34b an den Einschlagachsen 61a, 61b derart auf die Innenseite des Rückbereichs 24 eingeschlagen, dass die Materialabschnitte 34a, 34b auf Stoß oder mit einem geringen Abstand voneinander, jedenfalls nicht einander überlappend zu liegen kommen (Figur 2a).

In einer alternativen Ausführungsform ist es jedoch denkbar und vorteilhaft, die in Längsrichtung aufeinander gefalteten hinteren Materialabschnitte 34a, 34b an den Einschlagachsen 61a, 61b derart weit auf die Innenseite des Rückbereichs 24 einzuschlagen, dass die Materialabschnitte 34a, 34b zumindest bereichsweise einander überlappend zu liegen kommen.

Der Inkontinenzartikel 9 wird nach Fertigstellung der Längsfaltung der hinteren und vorderen Materialabschnitte und nach dem Einschlagen der gefalteten Materialabschnitte auf die Innenseite des Hauptteils vorzugsweise mindestens einmal, vorzugsweise zweimal nach innen, vorzugsweise an in Querrichtung 38 verlaufenden Faltlinien 70, 71 (Figur 1), auf sich selbst gefaltet, vorzugsweise derart, dass zunächst der Vorderbereich 22 nach innen auf die Innenseite 33 des Hauptteils 20 und anschließend der Rückbereich 24 auf den Vorderbereich 22 gefaltet wird. Dadurch entsteht ein im Herstell- und Verpackungsprozess aufgrund seiner kompakten Größe gut handhabbares Produkt, dessen Sichtseiten in der zusammengefalteten Konfiguration durch die auch im Anwendungszustand die äußere Lage bildende Materiallage 30 gebildet wird, so dass die Innenseite 33 des Artikels 9 vor Gebrauch vor Verunreinigung geschützt ist.

Anhand der Figur 1 sind die Abmessungen der Teilabschnitte 80b, 82b, 84b, 86b des Materialabschnitts 34b in der dargestellten Ausführungsform veranschaulicht. Die Abmessungen sind vorzugsweise identisch mit den Abmessungen der Teilabschnitte 80a, 82a, 84a, 86a des Materialabschnitts 34a. Die Gesamtbreite L1 in Querrichtung 38 eines am Hauptteil 20 angefügten, entfalteten vorderen und hinteren Materialabschnitts beträgt in Querrichtung 38 220 mm. Die Breite L2 des an den Hauptteil 20 angrenzenden Teilabschnitts 80b beträgt ca. 70 mm. Die Breite L3 des mittleren Teilabschnitts 82b und die Breite L4 des mittleren Teilabschnitts 84b beträgt ca. jeweils 40 mm, und die Breite L5 des äußeren, den Randbereich 37 umfassenden Teilabschnitts 86b beträgt ca. 70 mm. Die Breite des Bereiches, an dem der Materialabschnitt 34a, 34b überlappend an den Hauptteil 20 angefügt ist, beträgt jeweils ca. 25 mm bis 30 mm. Die Länge der Materialabschnitte im Vorderbereich L6 beträgt 230 mm, die Länge der Materialabschnitte im Rückbereich L7 beträgt 300 mm.

Bei Inkontinenzartikeln 9 der Konfektionsgrößen S (Small) und M (Medium) beträgt die Gesamtbreite L1 in Querrichtung 38 eines entfalteten vorderen und hinteren Materialabschnitts 140 mm bis 180 mm. Die Breite L2 des an den Hauptteil 20 angrenzenden Teilabschnitts 80b beträgt 20 mm bis 85 mm. Die Breite L3 des mittleren Teilabschnitts 82b und die Breite L4 des mittleren Teilabschnitts 84b betragen jeweils 20 mm bis 45 mm, und die Breite L5 des äußeren, den Randbereich 37 umfassenden Teilabschnitts 86b beträgt 50 mm bis 60 mm. Bei den Konfektionsgrößen L (Large) und XL (Extra Large) beträgt die Gesamtbreite L1 200 mm bis 265 mm, die Breite L2 beträgt 45 mm bis 125 mm, die Breite L3 und die Breite L4 betragen jeweils 35 mm bis 60 mm und die Breite L5 beträgt 55 mm bis 85 mm.

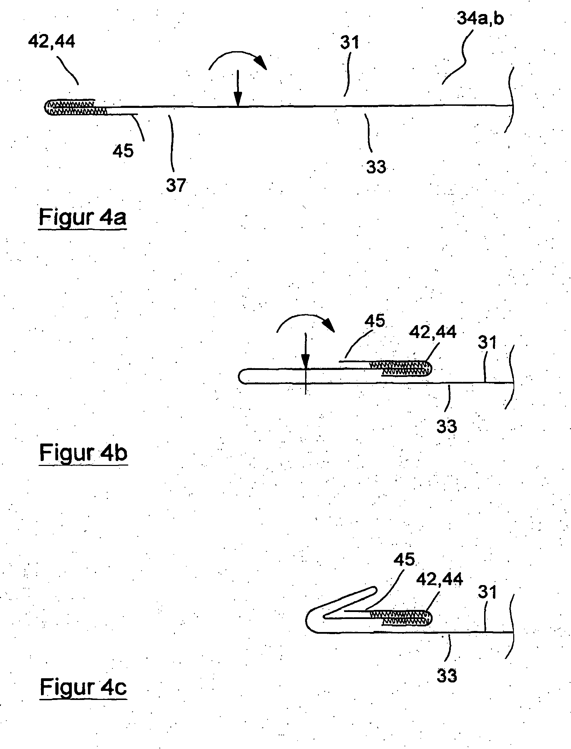

Figuren 4a, 4b und 4c veranschaulichen die Faltung des Materialabschnitts 34a, 34b auf sich selbst, wobei zunächst Außenseite 31 auf Außenseite 31gefaltet wird (Figur 4a) zu einer ersten gefalteten Anordnung (Figur 4b). Diese jetzt doppellagige Anordnung wird nochmals gefaltet, wobei nun Innenseite 33 auf Innenseite 33 zu liegen kommt (Figur 4c).

## Patentansprüche

1. Absorbierender Inkontinenzartikel (9) mit einem Hauptteil (20), bestehend aus einem Vorderbereich (22), einem Rückbereich (24) und einem in Längsrichtung (48) dazwischen liegenden zwischen den Beinen eines Benutzers zu liegen kommenden Schrittbereich (26), wobei der Hauptteil (20) einen Saugkörper (28) umfasst, und mit an einen Seitenrandabschnitt (36a, 36b) des Hauptteils (20) im Rückbereich (24) angefügten hinteren Materialabschnitten (34a, 34b) und mit an einen Seitenrandabschnitt (36c, 36d) des Hauptteils (20) im Vorderbereich (22) angefügten vorderen Materialabschnitten (35a, 35b), wobei sich die hinteren und vorderen Materialabschnitte (34a, 34b, 35a, 35b) in Querrichtung (38) über seitliche Längsränder (40a, 40b) des Hauptteils (20) hinaus erstrecken, wobei die hinteren und vorderen Materialabschnitte (34a, 34b, 35a, 35b) den Vorderbereich (22) und den Rückbereich (24) Im Anwendungszustand des Artikels (9) miteinander verbinden, wobei die hinteren und vorderen Materialabschnitte (34a, 34b, 35a, 35b) eine im Anwendungszustand auf der körperzugewandten Seite liegende Innenseite (33) und eine im Anwendungszustand auf der körperabgewandten Seite liegende Außenseite (31) aufweisen und wobei hintere Materialabschnitte (34a, 34b) an einem äußeren Längsrandbereich (37) der Materialabschnitte (34a, 34b) ein Verschlusselement (42) aufweisen, wobei das Verschlusselement (42) eine Verschlusselementlasche (44) mit einem freien Fingerlift (45) umfasst, welche vor Gebrauch auf die Innenseite (33) eines jeweiligen Materialabschnitts zurückgefaltet ist, wobei die hinteren Materialabschnitte (34a, 34b) vor Ingebrauchnahme des Artikels (9) auf sich selbst gefaltet sind an zu in Längsrichtung (48) parallel verlaufenden Falzlinien (50a, 50b; 52a, 52b, 54a, 54b) unter Bildung von Inneren Teilabschnitten (80a, 80b), mittleren Teilabschnitten (82a, 84a, 82b, 84b) und äußeren Teilabschnitten (86a, 86b), wobei die inneren Teilabschnitte (80a, 80b) an den jeweiligen Seitenrandabschnitt (36a, 36b) des Hauptteils (20) angefügt sind und die äußeren Teilabschnitte (86a, 86b) den Längsrandbereich (37) umfassen und die mittleren Teilabschnitte (82a, 84a, 82b, 84b) auf die Innenseite der äußeren Tellabschnitte (86a, 86b) gefaltet sind, derart dass die mittleren Teilabschnitte (82a, 84a, 82b, 84b) den freien Fingerlift (45) der Verschlusselementlasche (44) vollflächig überfangen und dass die derart gefalteten hinteren Materialabschnitte (34a, 34b) an Einschlagachsen (61a, 61 b) nach innen auf die Innenseite des Rückbereichs des Hauptteils (20) zu einer eingeschlagenen Anordnung eingeschlagen sind, wobei in der eingeschlagenen Anordnung die Innenseite des Randbereichs (37) von der Innenseite (33) des Rückbereichs (24) des Hauptteils (20) abgewandt orientiert ist.

2. Absorbierender Inkontinenzartikel (9) gemäß Anspruch 1, **dadurch gekennzeichnet, dass** die Verschlusselementlasche (44) zu mindestens 25 % und höchstens 90 % ihrer Erstreckung in Querrichtung (38), vorzugsweise zu mindestens 40 % und höchstens zu 75 % ihrer Erstreckung in Querrichtung (38) von mittleren Teilabschnitten (82a, 84a, 82b, 84b) überdeckt sind oder dass die mittleren Teilabschnitte (82a, 84a, 82b, 84b) die Verschlusselementlasche (44) um mindestens 10 mm und höchstens 50 mm, weiterhin bevorzugt mindestens 20 mm und höchstens 40 mm in Querrichtung (38) überdecken.

3. Absorbierender Inkontinenzartikel (9) gemäß Anspruch 1 oder 2, **dadurch gekennzeichnet, dass** ein äußerer Seitenrand (11) des äußeren Teilabschnitts (86a, 86b) eines hinteren Materialabschnitts (34a, 34b) gegenüber den darüber gefalteten mittleren Teilabschnitten (82a, 84a, 82b, 84b) in Querrichtung (38) um das Maß D übersteht.

4. Absorbierender Inkontinenzartikel (9) gemäß Anspruch 3, **dadurch gekennzeichnet, dass** das Maß D vorzugsweise mindestens 10 % und höchstens 85 %, bevorzugt mindestens 40 % und höchstens 60 %, der Breite L5 jedes äußeren Teilabschnittes (86a, 86b), weiterhin beträgt oder der Überstand in Querrichtung (38) um das Maß D vorzugsweise mindestens 5 mm und höchstens 50 mm, weiterhin bevorzugt mindestens 24 mm und höchstens 36 mm beträgt.

5. Absorbierender Inkontinenzartikel (9) gemäß einem der vorangehenden Ansprüche, **dadurch gekennzeichnet, dass** die Falzlinien die hinteren Materialabschnitte (34a, 34b) in jeweils vier Teilabschnitte (80a, 82a, 84a, 86a, 80b, 82b, 84b, 86b) unterteilen, wobei die Breiten L3, L4 der jeweilige mittleren Teilabschnitte(82a, 84a, 82b, 84b) kleiner sind als die Breiten L5 der jeweiligen äußeren Teilabschnitte (86a, 86b).

6. Absorbierender Inkontinenzartikel (9) gemäß einem der vorangehenden Ansprüche, **dadurch gekennzeichnet, dass** die Breiten L3, L4 der mittleren Teilabschnitte (82a, 84a, 82b, 84b) identisch sind und/oder das Verhältnis der Breiten L3, L4 der mittleren Teilabschnitte (82a, 84a, 82b, 84b) zu den Breiten L5 der äußeren Teilabschnitte (86a, 86b) zwischen 1:1 und 1: 2,5 liegt.

7. Absorbierender Inkontinenzartikel (9) gemäß einem der vorangehenden Ansprüche, **dadurch gekennzeichnet, dass** an jedem der hinteren Materialabschnitte (34a, 34b) an einem Längsrandbereich (37) ein Anfassbereich (58) vorhanden ist.

8. Absorbierender Inkontinenzartikel (9) gemäß einem der vorangehenden Ansprüche, **dadurch gekennzeichnet, dass** in Längsrichtung (48) aufeinander gefaltete hintere Materialabschnitte (34a, 34b) an den Einschlagachsen (61a, 61 b) derart auf die Innenseite des Rückbereich (24) eingeschlagen sind, dass die Materialabschnitte (34a, 34b) mit einem geringen Abstand voneinander oder auf Stoß zu liegen kommen oder zumindest bereichsweise einander überlappen.

9. Absorbierender Inkontinenzartikel (9) gemäß einem der vorangehenden Ansprüche, **dadurch gekennzeichnet, dass** die vorderen Materialabschnitte (35a, 35b) vor Ingebrauchnahme des zusammengefalteten Artikels (9) auf sich selbst gefaltet sind an zu der Längsrichtung (48) parallel verlaufenden Falzlinien (50a, 50b, 52a, 52b, 54a, 54b).

10. Absorbierender Inkontinenzartikel (9) gemäß einem der vorangehenden Ansprüche, **dadurch gekennzeichnet, dass** die vorderen Materialabschnitte (35a, 35b) vor Ingebrauchnahme des zusammengefalteten Artikels (9) auf sich selbst gefaltet sind an zu der Längsrichtung (48) parallel verlaufenden Falzlinien (50a, 50b, 52a, 52b, 54a, 54b) derart, dass die Falzlinien die vorderen Materialabschnitte (35a, 35b) in jeweils vier Teilabschnitte unterteilen, wobei die Breiten L3, L4 der mittleren Teilabschnitte (82a, 84a, 82b, 84b) kleiner sind als die Breite L5 der äußeren Teilabschnitte (86a, 86b).

11. Verfahren zur Herstellung eines gefalteten Inkontinerizartikels (9) gemäß einem der vorangehenden Ansprüche, **dadurch gekennzeichnet, dass** die hinteren Materialabschnitte (34a, 34b) zunächst einmal parallel zur Längsachse auf sich selbst gefaltet werden und anschließend die so gebildete doppellagige Anordnung nochmals parallel zur Längsachse auf sich selbst gefaltete wird und dabei der freie Fingerlift (45) der Verschlusselementlasche (44) von mittleren Teilabschnitten (82a, 84a, 82b, 84b) vollflächig überfangen wird zur Bildung einer zumindest bereichsweise vierlagigen Anordnung und dass die derart auf sich selbst gefalteten hinteren Materialabschnitte (34a, 34b) an den Einschlagachsen (61a, 61b) nach innen auf die Innenseite des Rückbereichs des Hauptteils (20) eingeschlagen werden, derart, dass die Innenseite des Längsrandbereichs (37) eines jeweiligen hinteren Materialabschnitts von der Innenseite (33) des Rückbereichs (24) des Hauptteils (20) abgewandt orientiert ist.

12. Verfahren zur Herstellung gefalteter Inkontinenzartikels (9) gemäß Anspruch 11, **dadurch gekennzeichnet, dass** die Förderung der Inkontinenzartikel (9) in einer Fertigungsmaschine parallel zur Längsrichtung (48) mit einer Bahngeschwindigkeit von mehr als 200 m/min, insbesondere mehr als 250 m/min, weiter insbesondere von mehr als 300 m/min, weiter insbesondere von mehr als 350 m/min erfolgt.

13. Verfahren zur Herstellung gefalteter Inkontinenzartikel (9) gemäß Anspruch 11 oder 12, **dadurch gekennzeichnet, dass** die Inkontinenzartikel (9) mindestens einmal, vorzugsweise zweimal nach innen, vorzugsweise an in Querrichtung (38) verlaufenden Faltlinien (70, 71), auf sich selbst gefaltet werden, vorzugsweise derart, dass zunächst der Vorderbereich (22) auf die Innenseite des Hauptteils und anschließend der Rückbereich (24) auf die Außenseite des Vorderbereichs gefaltet wird.

## Claims

1. Absorbent incontinence article (9) with a main part (20), consisting of a front section (22), a back section (24) and a crotch section (26) which lies in the longitudinal direction (48) between the front and the back sections and is intended to be placed between the legs of a user, the main part (20) having an absorbent body (28), and with rear material sections (34a, 34b) attached to a lateral edge section (36a, 36b) in the back section (24) of the main part (20), and with front material sections (35a, 35b) attached to a lateral edge section (36c, 36d) in the front section (22) of the main part (20), the rear and front material sections (34a, 34b, 35a, 35b) extending in the transverse direction (38) beyond lateral longitudinal edges (40a, 40b) of the main part (20), the rear and front material sections (34a, 34b, 35a, 35b) connecting the front section (22) and the back section (24) to one another in the applied state of the article (9), the rear and front material sections (34a, 34b, 35a, 35b) having, in the applied state, on the side facing the body an inside (33) and, in the applied state, on the side facing away from the body an outside (31), and the rear material sections (34a, 34b) having a closing element (42) on an outer longitudinal edge section (37) of the material sections (34a, 34b), the closing element (42) comprising a closing element tape (44) with a free fingerlift tab (45) which is folded back onto the inside (33) of a respective material section prior to use, the rear material sections (34a, 34b) being folded onto themselves prior to use of the article (9) along fold lines (50a, 50b, 52a, 52b, 54a, 54b) extending parallel to the longitudinal direction (48) and forming inner segments (80a, 80b), central segments (82a, 84a, 82b, 84b) and outer segments (86a, 86b), the inner segments (80a, 80b) being attached to the respective lateral edge section (36a, 36b) of the main part (20) and the outer segments (86a, 86b) comprising the longitudinal edge section (37) and the central segments (82a, 84a, 82b, 84b) being folded onto the inside of the outer segments (86a, 86b) in such a way that the central segments (82a, 84a, 82b, 84b) fully cover the free fingerlift tab (45) of the closing element tape (44) and that the thus folded rear material sections (34a, 34b) are turned in inward along turning-in axes (61a, 61b) onto the inside of the back section of the main part (20) to form a turned-in arrangement, the inside of the edge section (37) in the turned-in arrangement being oriented facing away from the inside (33) of the back section (24) of the main part (20).

2. Absorbent incontinence article (9) according to Claim 1, **characterized in that** the closing element tape (44) is covered over at least 25% and at most 90% of its extent in the transverse direction (38), preferably over at least 40% and at most 75% of its extent in the transverse direction (3), by central segments (82a, 84a, 82b, 84b), or **in that** the central segments (82a, 84a, 82b, 84b) cover the closing element tape (44) over at least 10 mm and at most 50 mm, furthermore preferably over at least 20 mm and at most 40 mm in the transverse direction (38).

3. Absorbent incontinence article (9) according to Claim 1 or 2, **characterized in that** an outer lateral edge (11) of the outer segment (86a, 86b) of a rear material section (34a, 34b) projects in the transverse direction (38) beyond the central segments (82a, 84a, 82b, 84b) folded over said lateral edge by the dimension D.

4. Absorbent incontinence article (9) according to Claim 3, **characterized in that** the dimension D is preferably at least 10% and at most 85%, preferably at least 40% and at most 60%, of the width L5 of each outer segment (86a, 86b), or the projection in the transverse direction (38) by the dimension D is preferably at least 5 mm and at most 50 mm, furthermore preferably at least 24 mm and at most 36 mm.

5. Absorbent incontinence article (9) according to one of the preceding claims, **characterized in that** the fold lines divide the rear material sections (34a, 34b) into four segments each (80a, 82a, 84a, 86a, 80b, 82b, 84b, 86b), the widths L3, L4 of the respective central segments (82a, 84a, 82b, 84b) being smaller than the widths L5 of the respective outer segments (86a, 86b).

6. Absorbent incontinence article (9) according to one of the preceding claims, **characterized in that** the widths L3, L4 of the central segments (82a, 84a, 82b, 84b) are identical and/or the ratio of the widths L3, L4 of the central segments (82a, 84a, 82b, 84b) to the widths L5 of the outer segments (86a, 86b) is between 1:1 and 1:2.5.

7. Absorbent incontinence article (9) according to one of the preceding claims, **characterized in that** a gripping region (58) is provided on a longitudinal edge section (37) of each of the rear material sections (34a, 34b).

8. Absorbent incontinence article (9) according to one of the preceding claims, **characterized in that** rear material sections (34a, 34b) folded onto themselves in the longitudinal direction (48) are turned in along the turning-in axes (61a, 61b) onto the inside of the back section (24) in such a way that the material sections (34a, 34b) come to lie at a small distance from one another or abut one another or overlap one another at least regionally.

9. Absorbent incontinence article (9) according to one of the preceding claims, **characterized in that** the front material sections (35a, 35b) are folded onto themselves prior to use of the folded-together article (9) along fold lines (50a, 50b, 52a, 52b, 54a, 54b) extending parallel to the longitudinal direction (48).

10. Absorbent incontinence article (9) according to one of the preceding claims, **characterized in that** the front material sections (35a, 35b) are folded onto themselves prior to use of the folded-together article(9) along fold lines (50a, 50b, 52a, 52b, 54a, 54b) extending parallel to the longitudinal direction (48) in such a way that the fold lines divide the front material sections (35a, 35b) into four segments each, the widths L3, L4 of the central segments (82a, 84a, 82b, 84b) being smaller then the width L5 of the outer segments (86a, 86b).

11. Method for manufacturing a folded incontinence article (9) according to one of the preceding claims, **characterized in that** the rear material sections (34a, 34b) are first folded onto themselves parallel to the longitudinal axis and the thus formed double-layered arrangement is subsequently once again folded onto itself parallel to the longitudinal axis, the free fingerlift tab (45) of the closing element tape (44) being fully covered by the central segments (82a, 84a, 82b, 84b) to form an at least regionally four-layered arrangement, and **in that** the rear material sections (34a, 34b) thus folded onto themselves are turned in inward along the turning-in axes (61a, 61b) onto the inside of the back section of the main part (20), such that the inside of the longitudinal edge section (37) of a respective rear material section is oriented facing away from the inside (33) of the back section (24) of the main part (20).

12. Method for manufacturing folded incontinence articles (9) according to Claim 11, **characterized in that** the conveyance of the incontinence articles (9) in a production machine is carried out parallel to the longitudinal direction (48) at a web speed of more than 200 m/min, in particular more than 250 m/min, furthermore in particular more than 300 m/min, furthermore in particular more than 350 m/min.

13. Method for manufacturing folded incontinence articles (9) according to Claim 11 or 12, **characterized in that** the incontinence articles (9) are folded inward onto themselves at least once, preferably twice, preferably along fold lines (70, 71) extending in the transverse direction (38), preferably in such a way that the front section (22) is first folded onto the inside of the main part and subsequently the back section (24) is folded onto the outside of the front section.

## Revendications

1. Article absorbant pour incontinence (9) comportant une partie principale (20), constituée d'une région avant (22), d'une région arrière (24) et d'une région de fourche (26) située entre elles dans la direction longitudinale (48), venant se positionner entre les jambes d'un utilisateur, la partie principale (20) comprenant un corps absorbant (28), et comportant des sections de matériau arrière (34a, 34b) raccordées à une section de bord latéral (36a, 36b) de la partie principale (20) dans la région arrière (24) et comportant des sections de matériau avant (35a, 35b) raccordées à une section de bord latéral (36c, 36d) de la partie principale (20) dans la région avant (22), les sections de matériau arrière et avant (34a, 34b, 35a, 35b) s'étendant dans la direction transversale (38) au-delà de bords longitudinaux latéraux (40a, 40b) de la partie principale (20), les sections de matériau arrière et avant (34a, 34b, 35a, 35b) reliant l'une à l'autre la région avant (22) et la région arrière (24) dans l'état d'utilisation de l'article (9), les sections de matériau arrière et avant (34a, 34b, 35a, 35b) présentant un côté intérieur (33) situé du côté tourné vers le corps dans l'état d'utilisation et un côté extérieur (31) situé du côté opposé au corps dans l'état d'utilisation et les sections de matériau arrière (34a, 34b) présentant un élément de fermeture (42) au niveau d'une région de bord longitudinal extérieure (37) des sections de matériau (34a, 34b), l'élément de fermeture (42) comprenant une patte d'élément de fermeture (44) avec une languette libre Fingerlift (45), qui est repliée avant l'utilisation sur le côté intérieur (33) d'une section de matériau respective, les sections de matériau arrière (34a, 34b) étant repliées sur elles-mêmes avant l'utilisation de l'article (9) au niveau de lignes de pliage (50a, 50b, 52a, 52b, 54a, 54b) s'étendant parallèlement dans la direction longitudinale (48) en formant des sections partielles intérieures (80a, 80b), des sections partielles centrales (82a, 84a, 82b, 84b) et des sections partielles extérieures (86a, 86b), les sections partielles intérieures (80a, 80b) étant raccordées à la section de bord latéral respective (36a, 36b) de la partie principale (20) et les sections partielles extérieures (86a, 86b) comprenant la région de bord longitudinal (37) et les sections partielles centrales (82a, 84a, 82b, 84b) étant repliées sur le côté intérieur des sections partielles extérieures (86a, 86b), de telle sorte que les sections partielles centrales (82a, 84a, 82b, 84b) recouvrent sur toute leur surface la languette libre Fingerlift (45) de la patte d'élément de fermeture (44) et de telle sorte que les sections de matériau arrière ainsi pliées (34a, 34b) soient rabattues vers l'intérieur au niveau d'axes de rabattement (61a, 61b) sur le côté intérieur de la région arrière de la partie principale (20) pour produire un agencement rabattu, le côté intérieur de la région de bord (37), dans l'agencement rabattu, étant orienté à l'écart du côté intérieur (33) de la région arrière (24) de la partie principale (20).

2. Article absorbant pour incontinence (9) selon la revendication 1, **caractérisé en ce que** la patte d'élément de fermeture (44) est recouverte à raison d'au moins 25 % et d'au plus 90 % de son étendue dans la direction transversale (38), de préférence à raison d'au moins 40 % et d'au plus 75 % de son étendue dans la direction transversale (38), par des sections partielles centrales (82a, 84a, 82b, 84b) ou **en ce que** les sections partielles centrales (82a, 84a, 82b, 84b) recouvrent la patte d'élément de fermeture (44) sur au moins 10 mm et au plus 50 mm, de préférence en outre sur au moins 20 mm et au plus 40 mm dans la direction transversale (38).

3. Article absorbant pour incontinence (9) selon la revendication 1 ou 2, **caractérisé en ce qu'**un bord latéral extérieur (11) de la section partielle extérieure (86a, 86b) d'une section de matériau arrière (34a, 34b) dépasse d'une dimension D dans la direction transversale (38) par rapport aux sections partielles centrales pliées par-dessus (82a, 84a, 82b, 84b).

4. Article absorbant pour incontinence (9) selon la revendication 3, **caractérisé en ce que** la dimension D vaut de préférence au moins 10 % et au maximum 85 %, de préférence au moins 40 % et au maximum 60 %, de la largeur L5 de chaque section partielle extérieure (86a, 86b) ou le dépassement de la dimension D dans la direction transversale (38) vaut de préférence au moins 5 mm et au maximum 50 mm, et de préférence au moins 24 mm et au maximum 36 mm.

5. Article absorbant pour incontinence (9) selon l'une quelconque des revendications précédentes, **caractérisé en ce que** les lignes de pliage divisent les sections de matériau arrière (34a, 34b) en quatre sections partielles (80a, 82a, 84a, 86a, 80b, 82b, 84b, 86b) respectives, les largeurs L3, L4 des sections partielles centrales (82a, 84a, 82b, 84b) respectives étant inférieures aux largeurs L5 des sections partielles extérieures (86a, 86b) respectives.

6. Article absorbant pour incontinence (9) selon l'une quelconque des revendications précédentes, **caractérisé en ce que** les largeurs L3, L4 des sections partielles centrales (82a, 84a, 82b, 84b) sont identiques et/ou le rapport des largeurs L3, L4 des sections partielles centrales (82a, 84a, 82b, 84b) aux largeurs L5 des sections partielles extérieures (86a, 86b) est compris entre 1:1 et 1:2,5.

7. Article absorbant pour incontinence (9) selon l'une quelconque des revendications précédentes, **caractérisé en ce qu'**au niveau de chacune des sections de matériau arrière (34a, 34b), une région de saisie (58) est prévue au niveau d'une région de bord longitudinal (37).

8. Article absorbant pour incontinence (9) selon l'une quelconque des revendications précédentes, **caractérisé en ce que** des sections de matériau arrière (34a, 34b) pliées les unes sur les autres dans la direction longitudinale (48) sont rabattues au niveau des axes de rabattement (61a, 61b) de telle sorte sur le côté intérieur de la région arrière (24) que les sections de matériau (34a, 34b) viennent s'appliquer avec une légère distance entre elles ou en aboutement ou se chevauchent mutuellement au moins en partie.

9. Article absorbant pour incontinence (9) selon l'une quelconque des revendications précédentes, **caractérisé en ce que** les sections de matériau avant (35a, 35b), avant l'utilisation de l'article replié (9), sont repliées sur elles-mêmes au niveau de lignes de pliage (50a, 50b, 52a, 52b, 54a, 54b) s'étendant parallèlement à la direction longitudinale (48).

10. Article absorbant pour incontinence (9) selon l'une quelconque des revendications précédentes, **caractérisé en ce que** les sections de matériau avant (35a, 35b), avant l'utilisation de l'article replié (9), sont repliées sur elles-mêmes au niveau de lignes de pliage (50a, 50b, 52a, 52b, 54a, 54b) s'étendant parallèlement à la direction longitudinale (48), de telle sorte que les lignes de pliage divisent les sections de matériau avant (35a, 35b) en quatre sections partielles respectives, les largeurs L3, L4 des sections partielles centrales (82a, 84a, 82b, 84b) étant inférieures à la largeur L5 des sections partielles extérieures (86a, 86b).

11. Procédé de fabrication d'un article pour incontinence plié (9) selon l'une quelconque des revendications précédentes, **caractérisé en ce que** les sections de matériau arrière (34a, 34b) sont pliées sur elles-mêmes d'abord une fois parallèlement à l'axe longitudinal et ensuite l'agencement à deux couches ainsi formé est encore une fois replié sur lui-même parallèlement à l'axe longitudinal et en l'occurrence la languette libre Fingerlift (45) de la patte d'élément de fermeture (44) est recouverte sur toute sa surface par les sections partielles centrales (82a, 84a, 82b, 84b) pour former un agencement au moins en partie à quatre couches et **en ce que** les sections de matériau arrière (34a, 34b) pliées de telle sorte sur elles-mêmes sont rabattues au niveau des axes de rabattement (61a, 61b) vers l'intérieur sur le côté intérieur de la région arrière de la partie principale (20) de telle sorte que le côté intérieur de la région de bord longitudinal (37) d'une section de matériau arrière respective soit orienté de manière opposée au côté intérieur (33) de la région arrière (24) de la partie principale (20).

12. Procédé de fabrication d'articles pour incontinence pliés (9) selon la revendication 11, **caractérisé en ce que** le transport des articles pour incontinence (9) s'effectue dans une machine de fabrication parallèlement à la direction longitudinale (48) avec une vitesse de bande supérieure à 200 m/min, en particulier supérieure à 250 m/min, plus particulièrement supérieure à 300 m/min, et tout particulièrement supérieure à 350 m/min.

13. Procédé de fabrication d'articles pour incontinence pliés (9) selon la revendication 11 ou 12, **caractérisé en ce que** les articles pour incontinence (9) sont pliés sur eux-mêmes au moins une fois, de préférence deux fois, vers l'intérieur, de préférence au niveau de lignes de pliage (70, 71) s'étendant dans la direction transversale (38), de préférence de telle sorte que la région avant (22) soit d'abord pliée sur le côté intérieur de la partie principale et qu'ensuite la région arrière (24) soit pliée sur le côté extérieur de la région avant.
